# EUROPEAN PATENT SPECIFICATION

(11) **EP 2 212 421 B1**
(45) Date of publication and mention of the grant of the patent: **08.08.2012**
(21) Application number: 08849060.2
(22) Date of filing: 14.11.2008
(51) Int. Cl.: C12N 15/113

(54) **MIRNA, SIRNA AND USE THEREOF IN THERAPY**
MIRNA, SIRNA UND VERWENDUNG DAVON IN DER THERAPIE
ARNMI, ARNSI ET LEURS UTILISATIONS THÉRAPEUTIQUES

(30) Priority: 15.11.2007 IT RM20070595
(43) Date of publication of application: 04.08.2010
(73) Proprietor: Universita'Degli Studi di Roma "La Sapienza", 00185 Roma (IT)
(72) Inventor: BOZZONI, Irene, I-00185 Rome (IT); FATICA, Alessandro, I-00185 Rome (IT); ROSA, Alessandro, I-00185 Rome (IT)
(74) Representative: Capasso, Olga
(86) International application number: PCT/IB2008/054774
(87) International publication number: WO 2009/063426

(56) References cited:
- FAZI FRANCESCO ET AL: "A minicircuitry comprised of MicroRNA-223 and transcription factors NFI-A and C/EBP alpha regulates human granulopoiesis" CELL, vol. 123, no. 5, December 2005 (2005-12), pages 819-831, XP002520389 ISSN: 0092-8674
- NERVI C ET AL: "Emerging role for microRNAs in acute promyelocytic leukemia" CURRENT TOPICS IN MICROBIOLOGY AND IMMUNOLOGY SPRINGER-VERLAG BERLIN, HEIDELBERGER PLATZ 3, D-14197 BERLIN, GERMANY SERIES : CURRENT TOPICS IN MICROBIOLOGY AND IMMUNOLOGY (ISSN 0070-217X(PRINT)), 1 January 2007 (2007-01-01), pages 73-84, XP009114041 ISSN: 978-3-540-34592-3(H)
- KIM DANIEL H ET AL: "Strategies for silencing human disease using RNA interference" NATURE REVIEWS GENETICS, vol. 8, no. 3, March 2007 (2007-03), pages 173-184, XP002520391 ISSN: 1471-0056
- SCHERR MICHAELA ET AL: "Gene silencing by small regulatory RNAs in mammalian cells" CELL CYCLE, LANDES BIOSCIENCE, US, vol. 6, no. 4, 15 February 2007 (2007-02-15), pages 444-449, XP002502224 ISSN: 1551-4005
- CHUNG KWAN-HO ET AL: "Polycistronic RNA polymerase II expression vectors for RNA interference based on BIC/miR-155." NUCLEIC ACIDS RESEARCH 2006, vol. 34, no. 7, 2006, page e53, XP002520392 ISSN: 1362-4962

## Description

### FIELD OF THE INVENTION

The present invention relates to the combined use of microRNA (miRNA) and/or interfering RNA (siRNA) for inducing hematopoietic differentiation and for leukaemia therapy.

### STATE OF THE ART

Leukemia is a cancer of the blood or bone marrow and is characterized by an abnormal proliferation of blood cells, usually white blood cells (leukocytes).

Acute Myeloid Leukemia (AML) is a blood cell tumour of the myeloid line (erythrocytes, granulocytes, monocytes, macrophages, megakaryocytes and precursors thereof) characterized by a rapid proliferation of neoplastic (leukemic) cells that accumulate in the bone marrow, inhibiting the differentiation and the multiplication of normal blood cells (Tenen, 2003).

AML is the most common leukemic form in adults. The neoplastic cell in AML is called myeloblast. In normal hematopoiesis, myeloblast is a precursor that matures gradually in a cell of the myeloid line. In AML, myeloblast undergoes genetic changes which "freeze" the cell in an immature state, by preventing its differentiation. There are different AML subclasses which can differ largely therebetween. AML diversity and heterogeneity derive from the fact that leukemic transformation can take place during one of the several phases of myeloid cell differentiation. In many patients with AML, specific cytogenetic alterations, such as chromosomic translocations, characterizing a determined AML type, can be identified. Chromosomic translocation produces aberrant fusion proteins, usually transcription factors, whose altered properties cause or contribute to the differentiation blockade. For example, in the acute promyelocytic leukemia (APL), the translocation t(15;17) produces a fusion protein between PML (transcription factor) and the receptor of the retinoic acid (RARα), able to bind specific sequences onto the promoters of myeloid-specific genes recognized by RARα with a consequent inhibition of the myeloid differentiation. AMLs are classified according to the French-American-British system (FAB) based on the morphologic characterization of neoplastic cells observed under microscope and/or on specific cytogenetic analyses to underline the chromosomal abnormalities. The FAB system classifies AMLs into eight sub-types, from M0 to M7, depending on the cell from which the leukemic blast derives and its maturity level (M0, Undifferentiated; M1, undifferentiated Myeloblastic; M2, differentiated Myeloblastic; M3, Promyelocytic; M4, Myelo-monocytic; M5, Monocytic; M6, Erythroblastic; M7, Megakaryocytic).

Some sub-types of leukemia are resistant to all type of pharmacological treatment.

The "RNA interference" (RNAi) is a mechanism for regulating gene expression therethrough small interfering molecules of RNA (siRNA), typically long of approximately 21-23 nt, are able to inhibit the expression of specific messenger RNA (mRNA) containing nucleotidic sequences perfectly complementary to the siRNA (Scherr and Eder, 2007). MicroRNAs (miRNA) also belong to the class of non codifying small RNAs. They are produced by cell genes and they negatively regulate gene expression by means of transductional inhibition of the target mRNA (Bartel, 2004). They play crucial functions in the development, differentiation, proliferation, apoptosis and other important cell processes. Unlike siRNAs, which recognize sequences perfectly complementary on target mRNA and control the degradation thereof, miRNA recognize the target sequences which are localized in the non translated region at 3' (3'-UTR) of mRNAs, by means of imperfect matchings. Apart from these differences, siRNAs and miRNAs are similar in terms of molecular features, biogenesis and effector functions (Scherr and Eder, 2007). Similarly to siRNAs, miRNAs can also be administered to the cell as synthetic double-strand RNA (dsRNA), or produced by the cell with DNA expression cassettes.

The specificity and the efficiency of the RNAi mechanism make it a valid tool for inhibiting gene expression, both in cell cultures and in living organisms. Several studies have demonstrated that siRNAs are well tolerated and they have pharmacokinetic properties suitable for *in vivo* use (Kim and Rossi, 2007). For this reason there is the need for identifying siRNAs which can be pharmacological molecules.

As far as miRNAs are concerned, their usefulness in the treatment of specific diseases has not yet been demonstrated and, for the moment, their use is confined to the diagnosis of specific tumour classes. Moreover, cancer therapies based upon RNA have several advantages with respect to classic pharmacological therapies: they are versatile, thanks to a good prediction of the interaction between RNA and substrate to be inhibited; they are effective, as they act at early stages of gene expression (mRNA); they guarantee high specificity with low complexity, considering that molecules of about 20 nucleotides are sufficient to confer a specific interaction and at last, they show less toxicity with respect to conventional therapies against tumours.

Fazi et al (Cell, 2005, vol. 123, no. 5, p. 819-831) and Nervi et al (Current Topics in Microbiology and Immunology, 2007, 313, p. 73-84) disclose miRNA able to modulate hematopoietic differentiation and/or to act as oncosuppressor, and siRNA able to modulate hematopoietic differentiation or to inhibit the expression of a fusion product deriving from a chromosomic translocation associated to leukemia.

Kim et al (Nature Reviews Genetics, 2007, vol. 8, no. 3, p. 173-184) provides an overview of the therapeutic applications of RNAi in development.

Scherr et al (Cell Cycle, 2007, vol. 6, no. 4, p. 444-449) highlights the mechanism of RNAi and its activation, the use of plasmid-based and retro-lentiviral vector-based systems to mediate long-term RNAi, kinetic aspects of RNAi and their impact on selection of cell populations with modified gene expression.

### DESCRIPTION OF THE INVENTION

The present invention relates to the use of small RNAs, siRNAs and/or miRNAs for inducing hematopoietic differentiation and for the treatment of leukemia, for instance AMLs. In particular, siRNAs act against specific fusion products of chromosomic translocation or against known proteins to oppose specific hematopoietic differentiation pathways. miRNAs are specifically involved in the differentiation of hematopoietic precursors and/or able to oppose the neoplastic proliferation. The invention relates also to the combined use of miRNA and siRNA that represents an alternative therapeutic strategy to broad-spectrum drugs. This strategy is particularly effective for some sub-types of leukemia, resistant to any type of pharmacological treatment.

Various siRNAs are combined therebetween and/or with miRNAs with specific roles in hematopiesis (es. miR-223 and miR-424), or in neoplastic proliferation (es. miR-34; He et al., 2007). In several types of cancer a reduction in miRNAs levels is observed which can have an intrinsic function of tumoral suppressors- One of the miRNAs recently characterized as powerful tumoral suppressor in several types of tumours is miR-34 (He et al., 2007). Several studies have demonstrated miR-34 activation by the protein p53. In addition, it was also demonstrated that miR-34 activation can partially restore the action of p53 itself, in inducing the stopping of the cell cycle and in promoting apoptosis.

It is therefore an object of the invention a mixture of molecules comprising one miRNA and one siRNA for use in the treatment of leukemia, wherein the miRNA is able to modulate the granulocyte differentiation and is miR-223 and the siRNA is able to modulate hematopoietic differentiation and inhibits NFIA; siRNA against NFIA (siNFIA, NFIA accession number NM 005595).

In order to facilitate the co-expression of miRNAs and siRNAs, the authors have developed dicistronic expression cassettes under the control of the U1 promoter allowing the simultaneous expression of miRNA/siRNA pairs. In these vectors, the siRNA/miRNA pairs are cloned inside the dicistronic genes of human miRNAs, wherein the miRNA sequence is replaced by the one of interest. Such cassettes can be also used in lentiviral vectors for transduction experiments.

miRNA/siRNA expression has been examined with Northern analysis, the target gene suppression by Western analysis, whereas the differentiation level in absence of inducing agents has been analyzed by means of morphological and immunochemical analyses (Fazi et al., 2005). GFP gene existing in the lentiviral vector has furthermore allowed the isolation of infected cells.

Apart from the use of lentiviral vectors, whose genoma integrates into the DNA of the host cell, other episomal viral vectors (for example AAV) and synthetic RNA can also be used. In order to obtain a cell-specific administration, it is possible to use antibodies against specific antigens (for example CD33 for myeloid blasts) conjugated to synthetic RNAs.

Preferably the leukemia is an acute myeloid leukemia.

More preferably the medicament is in the form of nanocapsules.

It is a further object of the invention an expression vector able to express in a host cell simultaneously the miRNAs and siRNAs of the invention.

Preferably the sequences codifying for the miRNAs and for the siRNAs are under the control of the U1 RNA promoter.

Preferably the vector of the invention are for use in inducing hematopoietic differentiation or for gene therapy.

In the present invention the miRNAs or siRNA may be obtained by chemical synthesis or other means known to the skilled parson in the art.

The invention will be described in exemplifying examples with reference to the following figures:

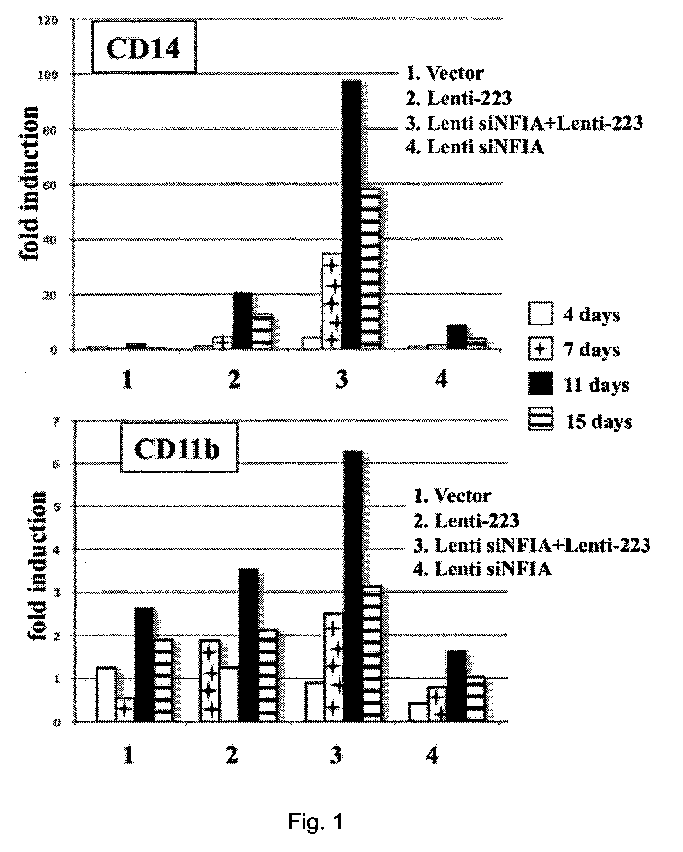

**Figure 1****.** NB4 cells were infected with lentiviral vectors expressing: 1, no small RNA (Vector); 2, miR-223 (Lenti-223); 4, siRNA against the "nuclear factor I-A", NFI-A (Lenti-siNFIA); 3, the combination of the two preceding ones. Markers CD 14 (A) and CD11b (B) onto the cells positive to GFP were analyzed after several days after the infection and analyzed with FACS; the reported values show the induction in the infected cells with respect to those not treated with the lentivirus.

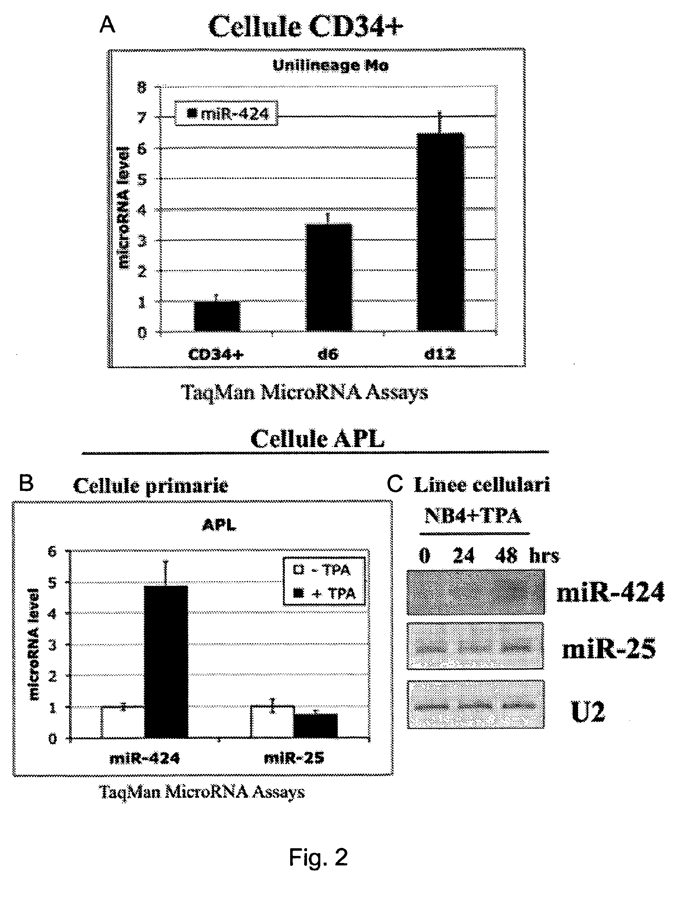

**Figure 2****.** Analysis of the miRNA levels by means of TaqMan MicroRNA Assays (Applied Biosystem) on: (A) total RNA from CD34+ cells induced to differentiate towards the monocyte line (samples taken at day 6 (d6) and day 12 (d12) as shown in the histogram), and (B) primary cells from APL patients before (-TPA) and after 48 hours of treatment with 12-O-Tetradecanoilphorbol 13-acetate (TPA), (+TPA). The probes used are shown. miR-25 is used as control. The histogram represents the average ± s.e.m from triplicates. (C) 10 µg of RNA, from NB4 cells not treated (lane 0) or treated with TPA for the designated time, were analyzed by Northern blot by using the indicated probes. snRNAs U2 and miR-25s were used as control.

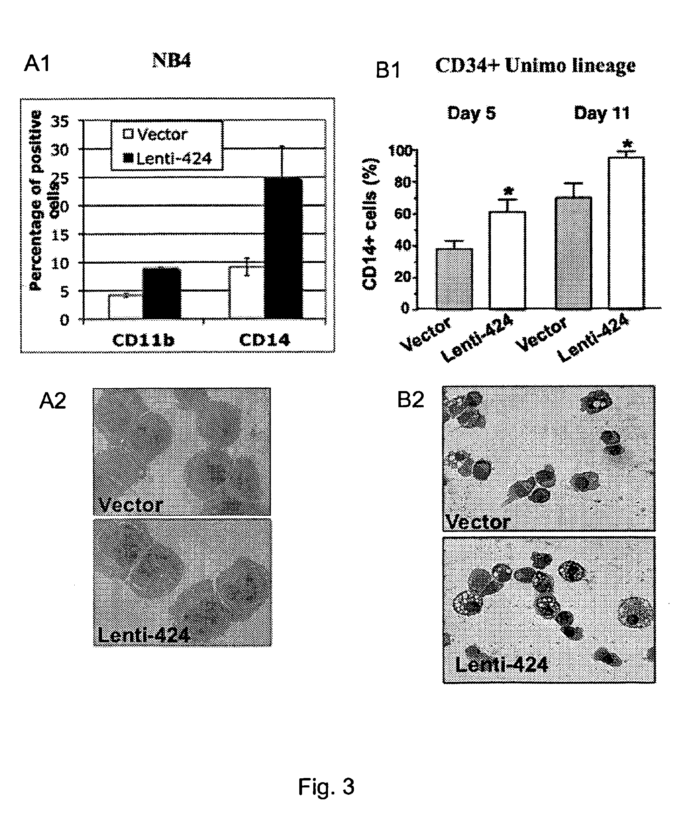

**Figure 3****.** (A1) NB4 cells were infected with the empty lentiviral vector (Vector) or with the lentivirus expressing miR-424 (Lenti-424) and incubated for 48 hours. The percentage of cells positive to CD11b or CD14 is shown. The histogram represents the average ± s.e.m from 3 replicates for each construct. (A2) The morphologic analysis underlines the differentiation of NB4 cells after 7 days of the infection with Lenti-424. (B1) CD34⁺ cells, induced to differentiate towards the monocyte line, were infected with Lenti-424 and purified by means of GFP expressed by the lentivirus. CD 14 expression as average ± s.e.m from 3 independent experiments is represented on the indicated days. (B2) The morphologic analysis shows an increase in the differentiation in cells treated with Lenti-424.

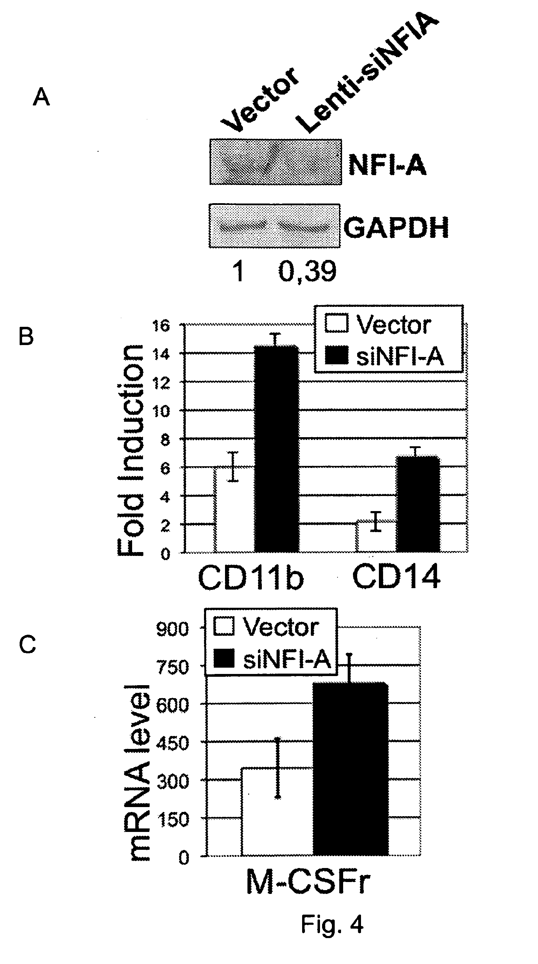

**Figure 4****.** NB4 cells were infected with the empty lentiviral vector (Vector) or with a lentiviral vector expressing siRNA against NFIA (Lenti-siNFIA). The proteins were extracted 48 hours after infection and 50 µg analyzed by means of Western blot with antibodies against NFI-A and GAPDH as control (A). The signals were normalized for GAPDH and the values, expressed as fractions with respect to the cells infected with the empty vector, are shown below the lanes. Cells positive to CD11b or CD 14 were analyzed with FACS (B). The mRNA expression of M-CSFr was measured by means of qRT-PCR (C). The values show the marker induction of cells treated with TPA for 48 hours with respect to non treated cells.

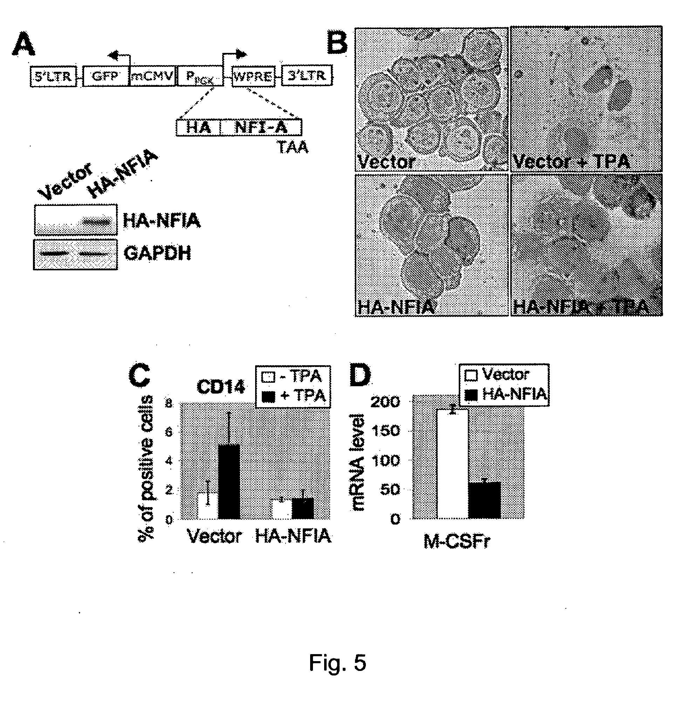

**Figura 5.** NFI-A overexpression. (A) Schematic representation of Lenti-HA-NFIA and analysis of their ectopic expression in NB4 cells by means of Western blot with anti-HA antibody. NB4 cells were infected with the empty vector (Vector) or with Lenti-HA-NFIA. After infection, half culture was treated with TPA (+TPA) and half without (-TPA). The cells were analyzed by Wright-Giemsa coloration for the morphology (B), by means of FACS for CD 14 expression (C) and qRT-PCR for M-CSFr expression (D). The histograms represent the average ± s.e.m. of triplicates.

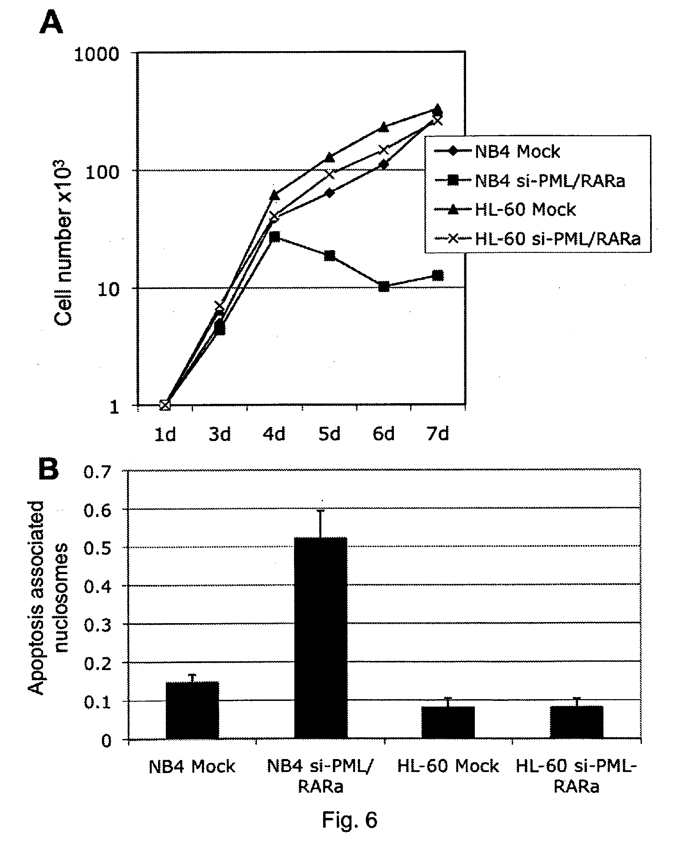

**Figura 6.** Knock-down of PML/RARα fusion gene blocks the growth of t(15;17) APL cell lines. NB4 cells bearing the t(15;17) (FAB subtype M3) responsible for the production of the PML/RARα fusion protein and HL-60 cells (FAB subtype M2) as control, were infected with lentivirus expressing siRNAs against the PML/RARα fusion protein (si-PML/RARα) or a control vector. (a) Growth curve of infected cells is shown. (b) Apoptosis rate 5 days after infection as measured by an assay based on cytoplasmic histone-associated DNA fragments (Cytodeath ELISA, Roche). Results present data from three replicates and are expressed as mean ± s.e.m.

### MATERIALS AND METHODS

### Vectors for microRNA and siRNA expression:

The snRNA U1 promoter and terminator were cloned inside the pSP65 vector (Stratagene), the KnpI and XhoI restriction sites were inserted between the promoter and the terminator. The human genomic sequences containing microRNA were cloned inside the U1 regulating elements in the KpnI and XhoI sites. The U1-microRNA expression cassettes were then amplified by PCR and cloned in the EcoRV site of the pRRLcPPT.hPGK.EGFP.WPRE lentiviral vector.

### Lenti-HA-NFIA

NFI-A was tagged to the N terminal with HA and cloned in the EcoRV and SalI sites of the pCCL.sin.cPPT.PGK.mCMV.GFP.WPRE lentiviral vector (Amendola et al., 2004) under the control of the PGK promoter and of the WPRE polyadenilation site.

### U1-miR-223

A fragment of 260 bp of human genomic sequence, containing miR-223 (nt. 143-164, underlined) was cloned between the snRNA U1 promoter and terminator. The pre-miR-223 is shown in bold (nt. 101-164) while the mature miR-223 is underlined. Flanking genomic sequences at 5' and 3' are indicated.

ATCATTCCTTTCTCTCTCTTTCCCTCTAGGGTCACATCTCCCAGGAAGAT 50
CTCACTTCCCCACAGAAGCTCTTGGCCTGGCCTCCTGCAGTGCCACGCTC 100
CGTGTATTTGACAAGCTGAGTTGGACACTCCATGTGGTAGAGTGTCAGTT 150
TGTCAAATACCCCAAGTGCGGCACATGCTTACCAGCTCTAGGCCAGGGCA 200
GATGGGATATGACGAATGGACTGCCAGCTGGATACAAGGATGCTCACCAA 250
GCACCAAGTT 260

(SEQ ID No. 1)

The pre-miR-223 structure is illustrated below (underlined the mature miR-223), :

    a     cc u                    gaguug        cau
gugcc cgcu   g guauuugacaagcu          gacacuc    g
||||| ||||   | ||||||||||||||          |||||||    u
cacgg guga   c cauaaacuguuuga          cugugag    g
    c     ac c                                aug

(SEQ ID No. 2)

Alternatively, the sequences flanking the pre-miR-223 (upstream and downstream the bold ones) can be replaced by other genomic sequences flanking other miRNAs. In particular, such other genomic sequences are the substrate for a highly efficient cut by the enzyme Drosha.

### pMC2

For siRNA production starting from the pre-miRNA of miR-223, the authors constructed a vector (pMC2) wherein the pre-miR-223 was replaced by a cassette (in bold) containing the restriction sites for the Bbs1 enzyme (underlined), leaving unaltered the flanking genomic sequences. The enzyme cuts are highlighted in the following scheme by arrows. 
↓ ↓
Bbs1
5′-...GCTCYYGCTTTCRRRRYYYRGAAGACRAAGTGCGG...-3′ (SEQ ID No.
3′-...CGAGRRCGAAAGYYYYRRRYCTTCTGYTTCACGCC...-5′ (SEQ ID No. 4)
↑ Bbs1 ↑
 wherein R=A or G and Y=C or T.

The cassette digested with BbsI allows inserting artificial pre-miRNAs obtained by means of matching synthetic oligonucleotides, obtained starting from the pre-miR-223 secondary structure.

The pre-miRNA structure (in bold) for the production of siRNA/miRNA (underlined) is illustrated in the following scheme (SEQ ID No. 5). 
a      NN N                    gaguug        cau
gugcc cgcu  N NNNNNNNNNNNNNN          NNNNcuc    g
||||| ||||  | ||||||||||||||          |||||||    u
cacgg guga  Y YY Y YY Y YY Y YY YY Y          Y YYYgag    g
c                                              aug
NN N
 wherein N=A, C, G or U and wherein when N = A, Y = U; when N = C, Y = G; when N = G,Y=C;whenN=U,Y=A.

### Scheme of oligonucleotides for cloning siRNA/miRNAs

GCTCNNNNNNNNNNNNNNNNNNNGAGTTGNNNNCTCCATGTGGTAGAGNNN
NNNNNNNNNNNNNNNNNNA

wherein N=A, C, G or T; 
Oligo antisense (SEQ ID No. 7):

CACTTNNNNNNNNNNNNNNNNNNNNNNNCTCTACCACATGGAGNNNNCAAC

TC NNNNNNNNNNNNNNNNN
 wherein N=A, C, G or T.

### U1-miR-424:

A fragment of 260 bp of human genomic sequence, containing miR-424 (nt. 92-113, underlined) was cloned between the snRNA U1 promoter and terminator.

GGGGTGGGGCGGGGCTTCCTTCAGTCATCCAGTCTTTATTCACCCGCAGG 50
TACCCCCAGATCGATCCCCCTTCATTGACTCCGAGGGATACAGCAGCAA 100
TTCATGTTTTGAAGTGTTCTAAATGGTTCAAAACGTGAGGCGCTGCTATA 150
CCCCCTCGTGGGAAGGTAGAAGGTGGGGTCTGCCGGACGCGTGTTCCTG 200
CCACCAGGTGCCCGCTCCCCGCGAGGCCGGCTCAGGAGCAGGTAGGTGGG 250
CGGGGGCTCG 260
(SEQ ID No. 8)

### U1-miR-34a:

A fragment of 260 bp of human genomic sequence, containing miR-34a (underlined) was cloned between the snRNA U1 promoter and terminator.

CATTTCCTTCTTATCAACAGGTGCTGGGGAGAGGCAGGACAGGCCTGTCC 50
CCCGAGTCCCCTCCGGATGCCGTGGACCGGCCAGCTGTGAGTGTTCTTT 100
GGCAGTGTCTTAGCTGGTTGTTGTGAGCAATAGTAAGGAAGCAATCAGCA 150
AGTATACTGCCCTAGAAGTGCTGCACGTTGTGGGCCCAAGAGGGAAGAT 200
GAAGCGAGAGATGCCCAGACCAGTGGGAGACGCCAGGACTTCGGAAGCTC 250
TTCTGCGCCA 260 (SEQ ID No. 9)

### Oligo sequences used for the specific siRNA production:

siRNA against NFIA: 5' -TGTTTTGACAGAAACTGACAG-3' (SEQ ID No. 10) or
5' -AACCAGAGGTCAAGCAGAA-3' (SEQ ID No. 11)
siRNA against PML/RARα: 5'-TCTCAATGGCTGCCTCCCCGGG-3' (SEQ ID No. 12)
siRNA against SUZ12: 5'-AGCTGTTACCAAGCTCCGTGA-3' (SEQ ID No. 13)
siRNA against AML1/ETO: 5'-CCUCGAAAUCGUACUGAGAAG-3' (SEQ ID No. 14)
siRNA against miR-223: 5'-TGGGGTATTTGACAAACTGACA-3' (SEQ ID No. 15)
siRNA against miR-424: 5'-TTCAAAACATGAATTGCTGCTG-3' (SEQ ID No. 16)

### Tandem miR-34/miR-223 sequence

The genomic sequences of miR-34a (in italic) and miR-223 were fused and cloned between U1 promoter and terminator.

CATTTCCTTCTTATCAACAGGTGCTGGGAGAGGCAGGACAGGCCTGTCC 50
CCCGAGTCCCCTCCGGATGCCGTGGACCGGCCAGCTGTGAGTGTTTCTTT 100
GGCAGTGTCTTAGCTGGTTGTTGTGAGCAATAGTAAGGAAGCAATCAGCA 150
AGTATACTGCCCTAGAAGTGCTGCACGTTGTGGGCCCAAGAGGGAAGAT 200
GAAGCGAGAGATGCCCAGACCAGTGGGAGACGCCAGGACTTCGGAAGCTC 250
TTCTGCGCCAATCATTCCTTTCTCTCTTTCCCTCTAGGGTCACATCTC 300
CCAGGAAGATCTCACTTCCCCACAGAAGCTCTTGGCCTGGCCTCCTGCAG 350
TGCCACGCTCCGTGTATTTGACAAGCTGAGTTGGACACTCCATGTGGTAG 400
AGTGTCAGTTTGTCAAATACCCCAAGTGCGGCACATGCTTACCAGCTCTA 450
GGCCAGGGCAGATGGGATATGACGAATGGACTGCCAGCTGGATACAAGGA 500
TGCTCACCAAGCACCAAGTT 520 (SEQ ID No. 17)

Tandem sequence for generic non coding RNA (microRNA and siRNA) expression

CATTTCCTTCTTATCAACAGGTGCTGGGAGAGGCAGGACAGGCCTGTCC 50
CCCGAGTCCCCTCCGGATGCCGTGGACCGGCCAGCTGTGAGTGTTTCTTT 100
NNNNNNNNNNNNNNNNNNNNNTGTGAGCAATAGTAAGGAAGNNNNNNNNN 150
NNNNNNNNNNNNNNNAGAAGTGCTGCACGTTGTGGGCCCAAGAGGGAAGAT 200
GAAGCGAGAGATGCCCAGACCAGTGGGAGACGCCAGGACTTCGGAAGCTC 250
TTCTGCGCCAATCATTCCTTTCTCTCTTTCCCTCTAGGGTCACATCTC 300
CCAGGAAGATCTCACTTCCCCACAGAAGCTCTTGGCCTGGCCTCCTGCAG 350

TGCCACGCTCNNNNNNNNNNNNNNNNNGAGTTGGNNNNTCCATGTGGTAG 400

AGNNNNNNNNNNNNNNNNNNNNNNAGTGCGGCACATGCTTACCAGCTCTA 450

GGCCAGGGCAGATGGGATATGACGAATGGACTGCCAGCTGGATACAAGGA 500

TGCTCACCAAGCACCAAGTT 520 (SEQ ID No. 18)

N = A, T, C or G represents the sequence of the specific miRNA or siRNA to express. This bicistronic vector allows the expression of any miRNA and siRNA combination. The miR-25 and snRNA U2 used control sequences are described in Fazi et al., 2005.

### Cell cultures, infections and immunophenotypic analyses

The NB4 cell line and CD34+ cells were kept in culture according to previously described standard protocols (see Fazi et al., 2005). The lentivirus preparation and infection were carried out as described (Fazi et al., 2005). The cell differentiation was analyzed by means of direct immunofluorescent colouration with specific antibodies and flow cytometry according to standard procedures (Fazi et al., 2005).

### RESULTS

### Induction of granulocyte differentiation by means of the combined use of miR-223 and siRNA against NFIA

The differential expression of miRNAs in the different stages of hematopoiesis suggests their role in the differentiation of blood cells (Geogantas et al., 2007). For miR-223, its induction during granulopoiesis was found in cells of Acute Promielocyte Leukemia (APL, Fazi et al., 2005). APLs result from the clonal expansion of hematopoietic progenitors blocked at the promyelocyte stage (sub-type M3) of differentiation.

NB4s are a cell line deriving from a APL patient which, if placed in culture with the appropriate differentiating agents, can differentiate in cells which morphologically and functionally ressemble a mature myeloid cell. It was also demonstrated by the authors of the invention that miR-223 stable expression in NB4 cells is able to increase the differentiation of these cells towards the granulocyte line, whereas miRNA functional inhibition inhibits the response of NB4 cells to granulocyte differentiation induced by retinoic acid. (Fazi et al., 2005). These results suggest a direct correlation between miR-223 levels and the maturity level of a myeloid precursor.

Upon studying the regulating loop wherein miR-223 is involved, the authors identified that mRNA for the NFIA transcriptional factor is regulated by miR-223 (Fazi et al., 2005). NFIA protein levels decrease during granulocyte differentiation and they follow an opposite course to miR-223 levels which on the contrary increase. Furthermore, siRNAs against NFIA increase the maturation towards the granulocyte line. Therefore, NFIA repression by miR-223 is an important event in the genetic programme which leads to the maturation of a myeloid precursor towards the granulocyte line.

The authors demonstrate a synergism between miR-223 overexpression and NFIA repression in granulocyte differentiation of APL cells. The authors used lentiviral constructs for the ectopic expression of miR-223 (Lenti-223) and siRNA against NFIA (lenti-siNFIA) in NB4 cells. In particular, a fragment of 260 base pairs of human genomic sequence containing miR-223 was cloned between the human snRNA U1 promoter, depending on RNA Pol II, and the specific terminator thereof. The sequence for the production of siRNA against NFIA was inserted inside the miR-223 precursor, by replacing the mature miRNA sequence with that of siRNA. The U1-ncRNA expression cassettes were then cloned in the lentiviral vector pRRLcPPT.hPGK.EGFP.WPRE (Fazi et al., 2005). The GFP gene existing inside the vector allowed to evaluate the infection efficiency during all experimental stages and to measure the differentiation markers only in the transduced cells. First, the authors compared the expression of granulocyte specific markers, such as CD11b and CD14, by means of flow cytometry. As shown in Figure 1, the infected cells both with Lenti-223 and Lenti-siNFIA show a higher expression of both markers than the cells infected with the single lentiviral constructs. The marginal increase noted with the empty vector, specially when CD11b is measured, is probably due to a non specific infection effect. These data demonstrate that the combined use of miRNA and siRNA stimulates efficiently myeloblast maturation.

Therefore, this synergic strategy can be used advantageously for a therapy of leukemia, in particular of the Acute Myeloid Leukemia.

An increase in miR-223 expression and NFIA repression was also demonstrated in HL-60 leukemic cell line of the M2 sub-type (Fazi et al., 2005), suggesting the combined use of miR-223 and siRNA against NFIA also for this AML sub-class.

The present data suggest that the use of miR-223 and siNFIA can be extended also to other AML type, independently from the leukemia genetic alteration, in particular to M0, M1, M2 and M4 sub-types which keep the potentiality of maturing in granulocytes.

### Induction of the monocyte differentiation by means of the combined use of miR-424 and siRNA against NFIA

Apart from maturing in granulocytes, AMLs of the M2 and M3 sub-types can be induced to differentiate in monocytes/magrophages by means of Vitamin D3 or 12-O-Tetradecanoilphorbol 13-acetate (TPA). The authors have identified a miRNA, miR-424, which is specifically induced during monocyte differentiation (Figure 2). The ectopic expression of miR-424 in NB4 and HL-60 cell lines and in CD34+ stem cells by means of transduction with lentivirus (see above) strongly stimulates maturation towards the monocyte/macrophage line (Figure 3). Furthermore, by using Lenti-siNFIA, the authors demonstrated that, as for the maturation towards the granulocyte line, NFIA repression also stimulates monocyte differentiation (Figure 4). The combined use of lentiviral constructs, for miR-424 ectopic expression and for producing siRNA against NFIA, thus represents an additional strategy for inducing differentiation of AML leukemic blasts. In particular, the use thereof can be extended to the M5 sub-type, wherein the blasts are blocked in a monoblastic stage of maturity such that they are not able to differentiate towards the granulocyte line any more.

Contrary to repression, NFIA overexpression contrasts significantly with monocyte differentiation (Figure 5) strengthening the proof that NFIA needs to be repressed by miR-223 and miR424 to allow the differentiation towards these two lineages. NFI-A overexpression can thus be used to unbalance myeloid differentiation towards the erythrocytic and megacaryocytic lines, wherein the levels of the transductional repressors NFIA, miR-223 and miR 424 are low.

### Induction of the myeloid differentiation by means of the combined use of miRNA and siRNA against fusion proteins and/or factors inhibiting the differentiation

The chromosomal translocations characterizing several AMLs usually produce the expression of oncogenic products, deriving from the fusion of: transcriptional factors with well defined roles in hematopoiesis (Rosenbauer and Tenen, 2007), such as AML1-ETO (t/8;21), CBF-MYH11 ("core-binding factor-myosin heavy chain" 11; inv16); MLL ("mixed lineage leukemia"; t11q23), and PML-RARα ("promyelocytic leukaemia-retinoic acid receptor α"; t/15;17). As a consequence, an aberrant transcriptional activity is present in the cell (Tenen, 2003; Rosenbauer and Tenen, 2007). For this reason, turning-off the activity of the fusion product by means of RNAi produces a therapeutic advantage. In particular, the authors used siRNA against:
i) AML1/ETO fusions for the AML therapy of M2 sub-type,
ii) PML-RARα fusions for the AML therapy of M3 sub-type.
Furthermore, the authors have combined the ectopic expression of miRNA with siRNA against factors with a well defined role in the inhibition of hematopoietic maturation. For example siRNA against NFIA (Fazi et al., 2005) or siRNA against SUZ12 (Villa et al., 2007). SUZ 12 is a factor involved in histone chemical modifications and its suppression by means of RNAi was shown to stimulate the differentiation of APL cells of the M3 sub-type (Villa et al., 2007).

### Efficacy of the activity of siRNA against the PML/Rarα fusion

In order to prove the efficacy of the siRNA against the fusion product PML/RARα, a NB4 cell line bearing the t(15;17) was infected with a lentivirus expressing siRNAs against PML/RARα (si-PML/RARα) or a control vector (mock). Another leukemia cell line without the t(15;17) translocation (HL-60) was utilized as control. Growth and apoptosis rate of infected cells was analysed at different days from infection. As shown in figure 6A, the si-PML/RARα specifically inhibited the growth of the NB4 cells without affecting the vitality of the HL-60. Moreover, the si-PML/RARα specifically increased the apoptosis rate of the cell line bearing the fusion PML/RARα product. These data indicate that the designed siRNA against PML/RARα is effective and specific.

### BIBLIOGRAPHY

Amendola M, et al. 2005 Nat Biotechnol 23:108-116.
Bartel, D. P. 2004, Cell, 23:281-297.
Georgantas, R.W. et al., 2007, PNAS, 20: 2750-2755.
Fazi, F., et al., 2005, Cell, 123: 819-831.
He, L., et al., 2007, Nature, 447:1130-1134.
Kim, D.H. et al., 2007, Nat. Rev. Genet. 8:173-184.
Rosenbauer, F., et al., 2007, Nat. Rev. Immunol. 7:105-117.
Scherr, M., Eder, M. 2007, Cell Cycle. 6: 444-449.
Tenen, D. G. 2003, Nat. Rev. Cancer 3: 89-101.
Villa, R., et al., 2007, Cancer Cell. 11: 513-525.

## Claims

1. A mixture of molecules comprising one miRNA and one siRNA for use in the treatment of leukemia, wherein the miRNA is able to modulate the granulocyte differentiation and is miR-223 and the siRNA is able to modulate hematopoietic differentiation and inhibits NFIA.

2. The mixture according to claim 1 wherein the leukemia is an acute myeloid leukemia.

3. The mixture according to any one of the preceding claims in the form of nanocapsules.

4. An expression vector able to express in a host cell simultaneously the miRNAs and the siRNA according to claim 1.

5. The vector according to claim 4 wherein the sequences codifying for the mRNA and for the siRNAs are under the control of the U1 RNA promoter.

6. The vector according to claim 4 or 5 for use in inducing hematopoietic differentiation or for use in gene therapy for the treatment of leukemia.

## Patentansprüche

1. Mischung von Molekülen, die eine miRNA und eine siRNA enthält, für die Verwendung in der Behandlung von Leukämie, wobei die miRNA in der Lage ist, die Granulozytendifferenzierung zu modulieren und miR-223 ist und die siRNA in der Lage ist, die hämatopoietische Differenzierung zu modulieren und NFIA zu inhibieren.

2. Mischung nach Anspruch 1, wobei die Leukämie eine akute myeloische Leukämie ist.

3. Mischung nach einem der vorangehenden Ansprüche in der Form von Nanokapseln.

4. Expressionsvektor, der in der Lage ist, in einer Wirtszelle die miRNA und die siRNA nach Anspruch 1 gleichzeitig zu exprimieren.

5. Vektor nach Anspruch 4, wobei die Sequenzen, welche für die miRNA und für die siRNA kodieren, unter der Kontrolle des U1-RNA-Promotors sind.

6. Vektor nach Anspruch 4 oder 5 für die Verwendung zur Induktion der hämatopoietischen Differenzierung oder zur Verwendung in der Gentherapie bei der Behandlung von Leukämie.

## Revendications

1. Mélange de molécules comprenant un ARNmi et un ARNsi pour une utilisation dans le traitement de la leucémie, dans lequel l'ARNmi est capable de moduler la différenciation des granulocytes et est miR-223 et l'ARNsi est capable de moduler la différenciation hématopoïétique et inhibe NFIA.

2. Mélange selon la revendication 1, dans lequel la leucémie est une leucémie aiguë myéloblastique.

3. Mélange selon l'une quelconque des revendications précédentes, sous forme de nanocapsules.

4. Vecteur d'expression capable d'exprimer simultanément, dans une cellule hôte, l'ARNmi et l'ARNsi selon la revendication 1.

5. Vecteur selon la revendication 4, dans lequel les séquences codant pour l'ARNmi et pour l'ARNsi sont sous le contrôle du promoteur d'ARN U1.

6. Vecteur selon la revendication 4 ou 5, pour une utilisation dans l'induction de la différenciation hématopoïétique ou pour une utilisation en thérapie génique pour le traitement de la leucémie.
